# FASCICULE DE BREVET EUROPEEN

(11) **EP 0 525 866 B1**
(45) Date de publication et mention de la délivrance du brevet: **05.03.1997**
(21) Numéro de dépôt: 92202189.4
(22) Date de dépôt: 17.07.1992
(51) Int. Cl.: C07D 333/32, C08F 134/04, H01B 1/12, C25B 3/02, H01L 51/00

(54) **Thiophènes fluorés, polymères dérivés de ces thiophènes, polymères conducteurs contenant ces polymères, procédés pour leur obtention, utilisations de ceux-ci et dispositifs contenant ces polymères conducteurs**
Fluorierte Thiophene, von diesen Thiophenen abgeleitete Polymere, leitfähige Polymere, die diese Polymere enthalten, Verfahren zu ihrer Herstellung, ihre Anwendung sowie Vorrichtungen die diese leitfähigen Polymere enthalten
Fluorinated thiophenes, polymers derived from these thiophenes, conducting polymers containing these polymers, methods for their preparation, their use and devices containing these conducting polymers

(30) Priorité: 29.07.1991 FR 9109715
(43) Date de publication de la demande: 03.02.1993
(73) Titulaire: SOLVAY (Société Anonyme), B-1050 Bruxelles (BE)
(72) Inventeur: Lemaire, Marc, F-69100 Villeurbanne (FR); Buechner, Werner, F-91600 Savigny s/Orge (FR); El Kassmi, Ahmed, F-69004 Lyon (FR); Hannecart, Etienne, B-3080 Tervuren (BE)
(74) Mandataire: Marckx, Frieda

(56) Documents cités:
- EP-A- 0 253 594
- EP-A- 0 328 984
- SYNTHETIC METALS vol. 26, no. 2, 1988, LAUSANNE, CH pages 153 - 168 R. M. BRYCE ET AL. 'Synthesis and cyclic voltammetric behavior of some 3-substituted thiophenes and pyrroles: precursors for the preparation of conducting polymers'

## Description

La présente invention concerne des thiophènes substitués par un groupement comportant au moins un atome de fluor. L'invention concerne également les polymères dérivés de ces thiophènes substitués ainsi que les polymères conducteurs d'électricité contenant ces polymères. L'invention concerne également des procédés pour l'obtention de ces thiophènes, de ces polymères et de ces polymères conducteurs d'électricité. L'invention concerne également les utilisations de ces thiophènes, de ces polymères et de ces polymères conducteurs d'électricité, ainsi que les dispositifs contenant les polymères et les polymères conducteurs d'électricité.

Des polymères conducteurs d'électricité dérivés de monomères de formule générale : 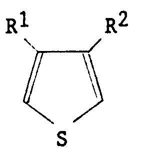 sont connus et ont notamment été décrits dans la demande de brevet européen EP 0253594 (Cookson Group) ; dans cette formule générale, R¹ peut représenter entre autres un groupement -O(CH₂)ₘNHCOR, -O(CH₂)ₘCONHR⁵ ou -(CH₂)ₙO(CHR³CH₂)ₚOR⁴ dans lequel R représente un groupement alkyle, R⁵ représente un groupement alkyle, aryle ou alkylaryle, R³ représente un atome d'hydrogène ou un groupement méthyle, R⁴ représente un groupement alkyle, m est un entier de 1 à 6, n est 0 ou un entier de 1 à 6, p est un entier de 1 à 6 et R² représente un atome d'hydrogène, un atome d'halogène ou un groupement aminé.

De même dans la demande de brevet européen 0328984 (Hoechst), on a décrit des polymères conducteurs d'électricité dérivés de monomères de formule générale : 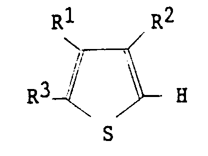 dans laquelle R¹ peut entre autres représenter un radical de formule -O(CH₂)ₙ-X, n étant un entier de 2 à 6 et X pouvant représenter un atome d'halogène, un hydroxyle ou un ester carboxylique, R² peut notamment représenter R¹, un atome d'hydrogène ou un groupement alkyle, R³ peut entre autres représenter un atome d'hydrogène.

Toutefois certaines applications électriques, telles que la réalisation de dispositifs (écrans d'affichage, commutateurs, éléments de mémoire,...) basés sur l'électrochromisme (impliquant une modification des propriétés d'absorption ou de transmission de la lumière du matériau mis en oeuvre, induite par une variation de la tension externe appliquée), d'électrodes de batteries rechargeables, de cellules photovoltaïques, de cellules électrochimiques, de dispositifs d'absorption des ondes électromagnétiques, de dispositifs utilisés en optique non linéaire, etc. exigent des polymères conducteurs aux propriétés particulières.

Ces propriétés particulières sont notamment la réversibilité électrochimique la plus complète et la stabilité la plus élevée possible du cycle d'oxydo-réduction entre les formes oxydée et réduite du système polymère-agent dopant, une variation importante des caractéristiques spectrales obtenues avec une variation de potentiel très faible, une bonne conductivité électrique, la possibilité d'offrir une large gamme de conductivité électrique, qui est en effet modulable selon l'utilisation souhaitée du système, et des absorptions importantes dans le domaine des rayonnements à haute fréquence.

La présente invention vise à fournir une nouvelle famille de thiophènes substitués permettant notamment d'obtenir des polymères conducteurs d'électricité qui présentent les propriétés particulières susmentionnées à un degré élevé.

L'invention concerne à cet effet des monomères dérivés de thiophènes substitués de formule générale : 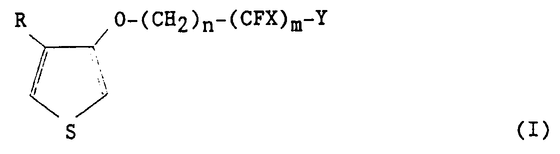 dans laquelle :
- R représente un atome d'hydrogène, un atome d'halogène, ou un groupement aliphatique contenant de 1 à 4 atomes de carbone,
- X représente un atome d'hydrogène ou un atome d'halogène,
- Y représente un atome d'hydrogène, un atome d'halogène, un groupement aliphatique linéaire ou non, non substitué ou substitué par un ou plusieurs atomes d'halogène et contenant de 1 à 6 atomes de carbone ou un groupement aromatique non substitué ou substitué par un ou plusieurs atomes d'halogène,
- n représente un nombre entier égal ou supérieur à 1,
- m représente un nombre entier égal ou supérieur à 1.
Habituellement :
- R représente un atome d'hydrogène, un atome de chlore, un atome de fluor ou un radical -CH₃,
- X représente un atome d'hydrogène ou un atome de fluor,
- Y représente :
   . un atome d'hydrogène, ou
   . un atome de fluor, ou
   . un radical phényle substitué au moins par un atome de fluor, un radical -CF₃, un groupement -CH₂F ou un groupement -CHF₂, ou
   . un groupement aliphatique linéaire, non substitué ou substitué par un ou plusieurs atomes de fluor et contenant de 1 à 4 atomes de carbone,
- n représente un nombre entier tel que 1 ≤ n ≤ 12,
- m représente un nombre entier tel que 1 ≤ m ≤ 18.
Généralement :
- R représente un atome d'hydrogène,
- X représente un atome d'hydrogène ou un atome de fluor,
- Y représente :
   . un atome d'hydrogène ou un atome de fluor ou
   . un groupement aliphatique linéaire, non substitué ou substitué par un ou plusieurs atomes de fluor et contenant de 1 à 4 atomes de carbone,
- n représente un nombre entier tel que 1 ≤ n ≤ 10,
- m représente un nombre entier tel que 1 ≤ m ≤ 16.
De manière préférée :
- R représente un atome d'hydrogène,
- X représente un atome de fluor,
- Y représente un atome d'hydrogène, un atome de fluor ou un radical -CF₃,
- n représente un nombre entier tel que 1 ≤ n ≤ 6,
- m représente un nombre entier tel que 1 ≤ m ≤ 14.
De manière particulièrement préférée :
- R représente un atome d'hydrogène,
- X représente un atome de fluor,
- Y représente un atome de fluor ou un radical -CF₃,
- n représente un nombre entier tel que 1 ≤ n ≤ 3,
- m représente un nombre entier tel que 1 ≤ m ≤ 12.
De bons résultats ont été obtenus lorsque :
- R représente un atome d'hydrogène,
- X représente un atome de fluor,
- Y représente un atome de fluor,
- n représente un nombre entier égal à 1,
- m représente un nombre entier égal à 1, 3 ou 7.

Les produits préferés sont l'oxyde de 2,2,2-trifluoréthyle et de 3-thiényle, l'oxyde de 4,4,4,3,3,2,2-heptafluoro-n-butyle et de 3-thiényle, l'oxyde de 8,8,8,7,7,6,6,5,5,4,4,3,3,2,2-pentadécafluoro-n-octyle et de 3-thiényle.

Les thiophènes substitués selon l'invention peuvent être synthétisés selon diverses méthodes, telle que notamment les méthodes selon les schémas de réaction ci-après.

Une réaction ayant donné de bons résultats consiste à faire réagir les composés selon le schéma suivant : schéma A

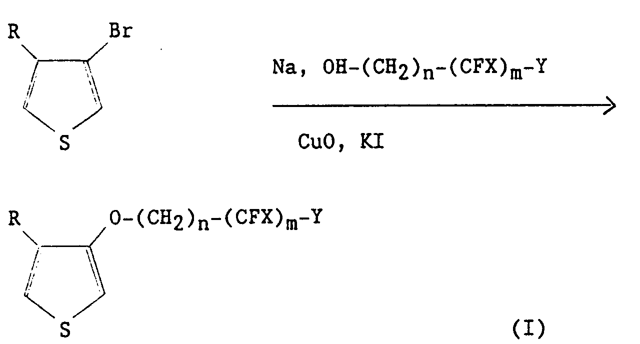

Le principe de réaction selon ce schéma A a été notamment décrit par S Gronowitz (Arkiv for Kemi Band 12, n° 25, 15 novembre 1957).

Le procédé qui répond à ce schéma A comprend les étapes suivantes :
- lors d'une première étape, on met en contact dans un réacteur le composé de formule générale OH-(CH₂)ₙ-(CFX)ₘ-Y, dans lequel n, m, X et Y sont définis ci-dessus, et du sodium, pour obtenir une suspension,
- lors d'une deuxième étape, on introduit dans la suspension obtenue du bromo-3 thiophène éventuellement substitué par le radical R, du CuO et du KI, pour obtenir un mélange,
- lors d'une troisième étape, on chauffe le mélange obtenu et on ajoute du KI, pour obtenir le monomère,
- lors d'une quatrième étape, le monomère obtenu est lavé et séparé.

Une variante de ce procédé consiste à introduire dans le réacteur directement le composé de formule générale Na⁺ [O-(CH₂)ₙ-(CFX)ₘ-Y]⁻, dans lequel n, m, X et Y sont définis ci-dessus.

Une autre réaction ayant donné de très bons résultats consiste à faire réagir les composés selon le schéma suivant : schéma B

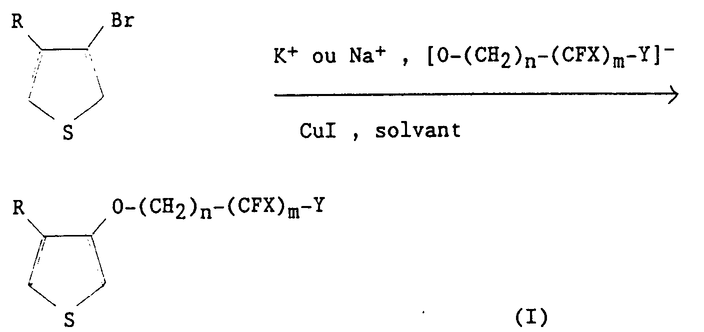

Le procédé qui répond à ce schéma B comprend les étapes suivantes :
- lors d'une première étape, on met en contact dans un réacteur le composé de formule générale OH-(CH₂)ₙ-(CFX)ₘ-Y, dans lequel n, m, X et Y sont définis ci-dessus, et un solvant de ce composé pour obtenir une suspension,
- lors d'une deuxième étape, on ajoute à la suspension obtenue, sous atmosphère inerte, du terbutylate de potassium ou du NaH, pour obtenir un mélange,
- lors d'une troisième étape, on introduit, dans le mélange obtenu, du bromo-3 thiophène éventuellement substitué par le radical R et du CuI, pour obtenir le monomère,
- lors d'une quatrième étape, le monomère obtenu est lavé et séparé.

Le solvant mis en oeuvre lors de la première étape est généralement un solvant polaire aprotique. De préférence on met en oeuvre comme solvant le diméthylformamide (DMF) ou le diméthoxyéthane.

La température de la réaction lors de la deuxième étape est généralement d'au moins environ -20°C. Elle ne dépasse pas en général environ 60°C. Elle est de préférence d'environ -10 à 40°C.

La température de la réaction lors de la troisième étape est généralement d'au moins environ 50°C. Elle ne dépasse pas en général 200°C. Elle est de préférence comprise entre environ 60 et 150°C.

La pression à laquelle est réalisé le procédé est généralement d'environ 1 à 10 bars et de préférence elle est égale à la pression atmosphérique.

Comme atmosphère inerte on choisit de préférence l'azote ou l'argon.

Lors de la quatrième étape le monomère obtenu est habituellement préalablement refroidi à la température ambiante, puis éventuellement dilué avec du chlorure de méthylène ou de l'éther. La suspension alors obtenue est séparée par filtration ou centrifugation, de préférence par filtration, puis lavée avec de l'acide chlorhydrique, puis avec de l'eau ou uniquement avec de l'eau. Le monomère obtenu est alors purifié par évaporation et/ou distillation.

Une autre méthode de synthèse des thiophènes substitués selon l'invention consiste en une cyclisation comparable à celle mise en oeuvre lors de la synthèse du thiophène. On prépare les thiophènes substitués selon l'invention par cyclisation, en présence de soufre ou d'un composé soufré tel que le sulfure de sodium, d'un butane substitué ou d'un butène substitué de formule générale :

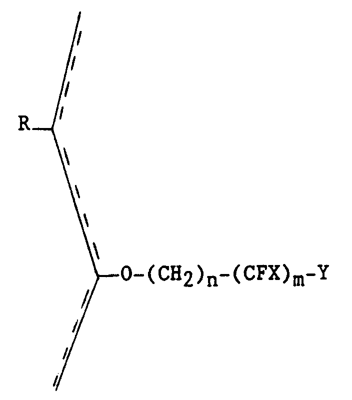

Un autre objet de l'invention est constitué par les polymères contenant des unités récurrentes dérivées de thiophène substitué selon l'invention.

L'invention concerne à cet effet des polymères substitués de formule générale : 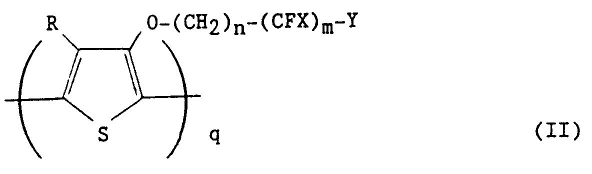 dans laquelle :
- q représente un nombre entier,
- R représente un atome d'hydrogène, un atome d'halogène, ou un groupement aliphatique contenant de 1 à 4 atomes de carbone,
- X représente un atome d'hydrogène ou un atome d'halogène,
- Y représente un atome d'hydrogène, un atome d'halogène, un groupement aliphatique linéaire ou non, non substitué ou substitué par un ou plusieurs atomes d'halogène et contenant de 1 à 6 atomes de carbone ou un groupement aromatique non substitué ou substitué par un ou plusieurs atomes d'halogène,
- n représente un nombre entier égal ou supérieur à 1,
- m représente un nombre entier égal ou supérieur à 1.
Habituellement :
- q représente un nombre entier compris entre 2 et 5000,
- R représente un atome d'hydrogène, un atome de chlore, un atome de fluor ou un radical -CH₃,
- X représente un atome d'hydrogène ou un atome de fluor,
- Y représente :
   . un atome d'hydrogène, ou
   . un atome de fluor, ou
   . un radical phényle substitué au moins par un atome de fluor, un radical -CF₃, un groupement -CH₂F ou un groupement -CHF₂, ou
   . un groupement aliphatique linéaire, non substitué ou substitué par un ou plusieurs atomes de fluor et contenant de 1 à 4 atomes de carbone,
- n représente un nombre entier tel que 1 ≤ n ≤ 12,
- m représente un nombre entier tel que 1 ≤ m ≤ 18.
Généralement :
- q représente un nombre entier compris entre 2 et 3000,
- R représente un atome d'hydrogène,
- X représente un atome d'hydrogène ou un atome de fluor,
- Y représente :
   . un atome d'hydrogène ou un atome de fluor ou
   . un groupement aliphatique linéaire, non substitué ou substitué par un ou plusieurs atomes de fluor et contenant de 1 à 4 atomes de carbone,
- n représente un nombre entier tel que 1 ≤ n ≤ 10,
- m représente un nombre entier tel que 1 ≤ m ≤ 16.
De manière préférée :
- q représente un nombre entier compris entre 2 et 1000,
- R représente un,atome d'hydrogène,
- X représente un atome de fluor,
- Y représente un atome d'hydrogène, un atome de fluor ou un radical -CF₃,
- n représente un nombre entier tel que 1 ≤ n ≤ 6,
- m représente un nombre entier tel que 1 ≤ m ≤ 14.
De manière particulièrement préférée :
- q représente un nombre entier compris entre 2 et 500,
- R représente un atome d'hydrogène,
- X représente un atome de fluor,
- Y représente un atome de fluor ou un radical -CF₃,
- n représente un nombre entier tel que 1 ≤ n ≤ 3,
- m représente un nombre entier tel que 1 ≤ m ≤ 12.
De bons résultats ont été obtenus lorsque :
- q représente un nombre entier compris entre 2 et 500,
- R représente un atome d'hydrogène,
- X représente un atome de fluor,
- Y représente un atome de fluor,
- n représente un nombre entier égal à 1,
- m représente un nombre entier égal à 1, 3 ou 7.

Les polymères préférés sont les polymères de l'oxyde de 2,2,2-trifluoréthyle et de 3-thiényle, les polymères de l'oxyde de 4,4,4,3,3,2,2-heptafluoro-n-butyle et de 3-thiényle, les polymères de l'oxyde de 8,8,8,7,7,6,6,5,5,4,4,3,3,2,2-pentadécafluoro-n-octyle et de 3-thiényle.

Les polymères substitués selon l'invention peuvent être synthétisés selon diverses méthodes, telles que notamment par polymérisation des thiophènes substitués définis ci-dessus ou par dédopage des polymères conducteurs d'électricité définis ci-après.

Un autre objet de l'invention est constitué par les polymères conducteurs d'électricité contenant un polymère substitué défini ci-dessus et un agent dopant.

L'agent dopant peut être un anion ou un cation, tel que défini ci-après.

La préparation des polymères conducteurs d'électricité selon l'invention peut s'effectuer par voie chimique ou par voie électrochimique.

La présente invention concerne également un procédé pour la préparation de polymères conducteurs d'électricité à base de polythiophènes substitués par polymérisation chimique du thiophène substitué dans un milieu réactionnel comprenant un oxydant et un solvant du thiophène substitué.

Comme oxydant on entend généralement un sel ferrique qui joue le rôle d'agent dopant. Généralement on utilise un sel ferrique organique ou inorganique. Habituellement on met en oeuvre un sel ferrique inorganique tel qu'un chlorure, un sulfate ou un nitrate. De manière préférée on met en oeuvre le chlorure ferrique. De manière particulièrement préférée on met en oeuvre le chlorure ferrique anhydre.

Le rapport molaire entre l'oxydant et le thiophène substitué est généralement d'au moins 1. Il ne dépasse pas en général 25. Il est de préférence compris entre 2 et 12.

Comme solvant du thiophène substitué on entend généralement un halogénure d'alkyle comprenant de 1 à 12 atomes de carbone. Habituellement on met en oeuvre un halogénure d'alkyle linéaire ou ramifié contenant de 1 à 8 atomes de carbone, l'halogénure étant un chlorure ou un fluorure. De préférence on met en oeuvre un chlorure d'alkyle linéaire contenant de 1 à 4 atomes de carbone. De manière particulièrement préférée on met en oeuvre le chloroforme ou le chlorure de méthylène.

La quantité de solvant mise en oeuvre dans le procédé selon l'invention est généralement d'au moins 0,5 par g de thiophène substitué. Elle ne dépasse pas en général 1000 ml par g de thiophène substitué. Elle est habituellement comprise entre 1 et 500 ml et de préférence entre 2 et 200 ml par g de thiophène substitué.

La réaction de polymérisation chimique est habituellement conduite sous atmosphère d'azote ou d'air sec et, de préférence, sous atmosphère d'azote.

La température à laquelle est réalisé le procédé est généralement d'au moins -40°C. Elle ne dépasse pas en général 50°C. Elle est habituellement comprise entre -30 et 40°C et de préférence entre -25 et 30°C lorsqu'on travaille à pression atmosphérique.

La pression à laquelle est réalisé le procédé est généralement d'environ 1 à 10 bars. De préférence elle est égale à la pression atmosphérique.

Le procédé selon l'invention peut avantageusement être réalisé en suivant les étapes :
- lors d'une première étape on introduit dans le réacteur sous atmosphère d'azote une fraction de la quantité de solvant et l'oxydant,
- lors d'une deuxième étape, on ajoute le thiophène substitué et une fraction de la quantité de solvant, avec obtention d'un polymère,
- lors d'une troisième étape, le polymère obtenu est séparé, lavé puis séché.

Lors de la troisième étape le polymère est séparé par filtration ou centrifugation. Le polymère est lavé avec un solvant. Il est lavé habituellement avec un halogénure d'alkyle tel que défini ci-dessus ou avec de l'acétonitrile, de préférence avec du chloroforme ou avec de l'acétonitrile.

Le procédé selon l'invention peut être réalisé dans tout appareillage ou tout réacteur permettant de réunir les conditions opératoires décrites ci-avant.

La présente invention concerne également un procédé pour la préparation de polymères conducteurs d'électricité à base de polythiophènes substitués par polymérisation électrochimique, généralement dans une cellule d'électrolyse, par oxydation anodique du thiophène substitué au sein d'un solvant polaire et en présence d'électrolytes appropriés suivant des techniques conventionnelles telles que notamment décrites dans la demande de brevet français 2 527 843.

La concentration en thiophène substitué est généralement d'au moins environ 10⁻³ mole par litre de solvant. Elle ne dépasse pas en général 1 mole par litre de solvant.

la température à laquelle est réalisé le procédé est généralement d'au moins 0°C. Elle ne dépasse pas en général 50°C. Elle est de préférence comprise entre 5 et 40°C.

La pression à laquelle est réalisé le procédé est généralement d'environ 1 à 5 bars. Elle est de préférence égale à la pression atmosphérique.

Comme solvant on utilise des solvants polaires possédant des propriétés dissolvantes à la fois vis-à-vis du thiophène substitué et de l'électrolyte choisi et stables dans le domaine des potentiels appliqués. Des exemples de solvants utilisables sont l'acétonitrile, le chlorure de méthylène, le nitrobenzène et le carbonate de propylène.

Les électrolytes sont généralement choisis parmi les sels conducteurs de formule C⁺A⁻ dans laquelle C⁺ est un cation et A⁻ est un anion.

Le cation C⁺ est choisi de préférence parmi les ions alcalins, les ions R₄N⁺ et R₄P⁺, R étant un radical alkyle, tel que les radicaux éthyle et butyle.

L'anion A⁻ est choisi de préférence parmi les ions ClO₄⁻, AsF₆⁻, SbF₆⁻,C₆H₅SO₃⁻, BF₄⁻, PF₆⁻ et CF₃SO₃⁻.

Des électrolytes typiques sont par exemple les fluorophosphates, tels que l'hexafluorophosphate de tétrabutylammonium, les fluoroborates, tels que le tétrafluoroborate de tétraéthylammonium et les perchlorates, tels que le perchlorate de lithium et le perchlorate de tétrabutylammonium.

La concentration en électrolyte est généralement d'au moins environ 10⁻³ mole par litre de solvant. Elle ne dépasse pas en général 1 mole par litre de solvant.

La cellule électrochimique au sein de laquelle peut s'effectuer la polymérisation des thiophènes substituès selon l'invention peut fonctionner dans des conditions potentiostatiques ou galvanostatiques.

Dans le cas potentiostatique, la cellule comprend, outre la source de courant externe, trois électrodes dont une électrode de référence de contrôle du potentiel.

Au cours de l'électrolyse une couche de polymère se dépose sur l'élément conducteur utilisé comme anode de la cellule d'électrolyse. Cette anode peut être réalisée en un métal noble, tel que l'or ou le platine, ou en un autre métal, tel que le cuivre, doré ou platiné, le titane, le nickel ou en un verre conducteur (oxyde d'étain, oxydes d'indium-étain). Après l'électrolyse, on dispose d'une électrode constituée par un corps conducteur enrobé par un film de polymère y adhérant et qui contient une certaine proportion de l'anion provenant de l'électrolyte. Le polymère et l'anion forment ainsi un complexe à transfert de charges. La composition chimique du film de polymère peut être représentée par la formule (M⁺A_{y}⁻)_{q} où M⁺ représent le thiophène substitué (monomère), A⁻ l'anion ou contre-ion, y la proportion en anion dans le polymère exprimée par unité monomérique (c'est-à-dire le taux de dopage) qui, dans le cas des polymères selon l'invention peut atteindre la valeur de 0,3, et q le degré de polymérisation.

Selon le procédé décrit ci-dessus, la polymérisation électrochimique du thiophène substitué s'effectue sur l'anode de la cellule d'électrolyse.

Pour obtenir une cathode recouverte d'un polymère dopé par des cations, on se sert de l'anode obtenue précédemment et on lui fait subir une double réduction. Une première réduction électrochimique est possible juste après la polymérisation en laissant l'anode dans la cellule d'électrolyse et en provoquant la décharge de la cellule. Cette décharge provoque l'extraction des anions dopant le polymère. On peut ensuite effectuer une seconde réduction sous atmosphère inerte, soit par voie chimique, soit par voie électrochimique. La voie chimique consiste à immerger le polymère dans une solution contenant les cations désirés. La voie électrochimique consiste à placer l'électrode en tant que cathode dans une cellule d'électrolyse contenant en solution les cations désirés.

Les polymères substitués selon l'invention peuvent être synthétisés par dédopage des polymères conducteurs d'électricité définis ci-dessus. Les procédés de dédopage de polymères conducteurs sont connus. Un procédé ayant donné de bons résultats consiste à mélanger le polymère conducteur avec de l'eau ou avec un alcool, tel que notamment du méthanol, puis à filtrer la suspension obtenue, le produit est ensuite lavé avec de l'eau ou de l'alcool puis séché en vue d'obtenir un polymère dédopé. De bons résultats ont été obtenus avec de l'eau.

Les polymères dédopés peuvent être mis en solution dans des solvants organiques. Ils peuvent être appliqués sous forme de couches épaisses ou minces, au travers ou non de masques sérigraphiques, ou sous forme de films d'épaisseur inférieure au micron sur divers supports tels que verre, verre métallisé, métaux, textiles pour obtenir des films isolants qui sont adhérents et homogènes. Ces films peuvent être dopés, de nouveau, pour être rendus conducteurs à des niveaux variables et modulables de conductivité électrique.

Les polymères conducteurs selon l'invention présentent un ensemble de propriétés tout à fait remarquables qui sont principalement :
- une réversibilité et une stabilité excellentes du cycle d'oxydo-réduction entre leurs formes oxydées et réduites ;
- une variation importante des caractéristiques spectrales obtenue avec une variation de potentiel faible, ce qui rend intéressante et économique leur utilisation en tant que matériau électrochromique ;
- des absorptions importantes dans le domaine des rayonnements à haute fréquence ;
- une mise en oeuvre aisée suite à leur solubilité dans de nombreux solvants organiques, tels que notamment le tétrahydrofurane, l'acétone, l'acétonitrile, le méthanol, le diméthylformamide ;
- une compatibilité avec d'autres polymères tels que le polychlorure de vinyle, le polyéthylène, le polypropylène ;
- la possibilité de les appliquer sous forme de couches épaisses ou minces, au travers ou non de masques sérigraphiques, ou sous forme de films d'épaisseur inférieure au micron sur divers supports tels que verre, verre métallisé, métaux, textiles pour obtenir des films conducteurs, ceux-ci étant adhérents, homogènes et très conducteurs ou à conductivité ajustable ;
- la possibilité de les utiliser en optique non linéaire en tant qu'interrupteurs optiques.

Ces propriétés remarquables des polymères conducteurs selon l'invention les rendent particulièrement utilisables pour la réalisation de dispositifs électroconducteurs dont le principe de fonctionnement est basé sur ces propriétés et qui constituent également un objet de la présente invention.

A titre d'exemples non limitatifs de dispositifs électroconducteurs contenant des polymères conducteurs selon l'invention, on peut citer :
- les dispositifs électrochimiques de stockage d'énergie, tels que batteries d'accumulateurs et piles rechargeables ou non, dont les anodes (respectivement les cathodes) sont constituées d'électrodes revêtues de films desdits polymères dopés par des anions (respectivement des cations) ;
- les dispositifs électrochromiques basés sur la modification du spectre optique desdits polymères selon leur état d'oxydation, qui se manifeste lors des cycles d'oxydation et de réduction des films de polymères déposés sur les anodes (respectivement les cathodes) de ces dispositifs lors de la charge et de la décharge ; à titre d'exemples de dispositifs on peut citer les écrans d'affichage, les dispositifs optoélectroniques, les mémoires et commutateurs optiques ;
- les dispositifs d'absorption des ondes électromagnétiques (écrans, capots) ;
- les dispositifs permettant le contact entre les isolants et les conducteurs (dispositifs anti-claquage,..).

Les polymères conducteurs d'électricité selon l'invention peuvent être aisément dispersés dans des résines polymériques diverses. Les polymères conducteurs et leurs mélanges avec des polymères thermoplastiques tels que le chlorure de polyvinyle, le polyéthylène, le polypropylène et leurs copolymères dérivés notamment, peuvent être malaxés et pressés à chaud pour obtenir des plaques ou objets composites. Ces plaques ou objets composites présentant une bonne résistance mécanique, sont homogènes et lisses. Ces plaques ou objets composites peuvent notamment être utilisés lors de la fabrication de blindage électromagnétique.

Les plaques ou objets composites peuvent être chargés par des charges diverses telles que des fibres de verre, du noir de carbone, du carbonate de calcium ou des particules métalliques.

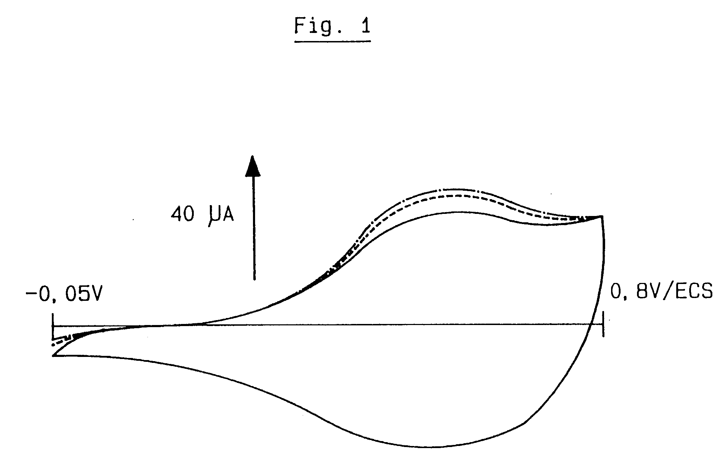

Les figures 1, 2 et 3 représentent un voltammogramme.

L'invention est illustrée par les exemples suivants.

### Exemple 1 : préparation de l'oxyde de 2,2,2-trifluoréthyle et de 3-thiényle

Comme réacteur on utilise un ballon de 250 ml, à trois cols, équipé d'un réfrigérant surmonté d'une garde de chlorure de calcium, d'un thermomètre, d'une admission d'argon, ce ballon est équipé d'une agitation magnétique de grande dimension et est placé dans un bain thermostatique.

Dans ce ballon, on introduit 20 ml (258 mmol.) de trifluoroéthanol CF₃CH₂OH et très lentement en 30 minutes environ 0,7 g (31mmol.) de sodium coupé en petits morceaux.

On chauffe cette suspension à 60°C pendant 30 minutes, puis on ramène la tempèrature à 20°C.

On introduit dans cette suspension 5 g (30,7 mmol.) de bromo-3 thiophène, 1,21 g (15 mmol.) de CuO et 0,076 g (0,46 mmol.) de KI.

Le ballon est maintenu à 100°C durant 4 jours, on introduit tous les jours dans ce ballon 50 mg de KI.

La suspension obtenue est lavée avec 200 ml d'eau, puis filtrée, extraite à l'éther et ensuite évaporée.

Le produit obtenu est purifié par distillation à 145-150°C.

On obtient 1,7 g de l'oxyde de 2,2,2-trifluoréthyle et de 3-thiényle.

Le rendement est de 30 %.

### Exemple 2 : préparation de l'oxyde de 2,2,2-trifluoréthyle et de 3-thiényle

Comme réacteur on utilise un ballon de 500 ml muni de trois cols et équipé d'une agitation, d'un thermomètre, d'une circulation d'azote et d'un réfrigérant.

Dans ce ballon on introduit 120 ml de diméthylformamide et 36 ml (475 mmol.) de trifluoroéthanol.

On ajoute ensuite 14,25 g (594 mmol.) de NaH dans le ballon lentement en 15 minutes environ, en refroidissant à l'aide d'un mélange de glace et d'eau et sous atmosphère inerte d'azote.

Le ballon est maintenu 15 minutes à 0°C.

Puis on ajoute 30,8 ml (330 mmol.) de bromo-3 thiophène et 1,25 g (6,6 mmol.) d'iodure cuivreux CuI dans le ballon.

Le ballon est maintenu à 110°C pendant 5 heures sous agitation vive et sous atmosphère inerte.

On refroidit ensuite la suspension obtenue à 20°C et on la dilue avec 300 ml de chlorure de méthylène.

On filtre la suspension diluée, on lave avec de l'acide chlorhydrique 0,1 N puis avec de l'eau distillée jusqu'à neutralité.

La phase organique est alors évaporée à pression atmosphèrique pour éliminer le solvant puis distillée.

On obtient 38,9 g de l'oxyde de 2,2,2-trifluoréthyle et de 3-thiényle.

Le rendement est de 65 %.

### Exemple 3 : préparation de l'oxyde de 4,4,4,3,3,2,2-heptafluoro-n-butyle et de 3-thiényle

Comme réacteur, on utilise un ballon de 100 ml muni de trois cols et équipé d'une agitation magnétique.

Dans ce ballon, on introduit 35 ml de diméthoxyéthane fraîchement distillé, puis 75,6 mmol. d'alcool polyfluoré CF₃(CF₂)₂CH₂OH.

Puis on introduit sous atmosphère d'azote 2,15 g (90,7 mmol.) d'hydrure de sodium.

On maintient la suspension obtenue 30 minutes sous agitation à 20°C.

On introduit ensuite dans le ballon 14 ml (150 mmol.) de bromo-3 thiophène, puis 7 g (37 mmol.) d'iodure cuivreux.

Le ballon est maintenu à 90°C pendant 15 heures.

Puis le ballon est refroidi à 20°C.

La suspension refroidie est filtrée, reprise à l'éther puis lavée à l'eau.

La phase organique est séchée et évaporée sous vide.

Le produit est purifié par distillation sous vide (18 mmHg).

On obtient l'oxyde de 4,4,4,3,3,2,2-heptafluoro-n-butyle et de 3-thiényle avec un rendement de 53 % par rapport à l'alcool polyfluoré.

### Exemple 4 : préparation de l'oxyde de 8,8,8,7,7,6,6,5,5,4,4,3,3,2,2-pentadécafluoro-n-octyle et de 3-thiényle

Comme réacteur on utilise un ballon de 100 ml muni de trois cols et équipé d'une agitation magnétique.

Dans ce ballon on introduit 35 ml de diméthoxyéthane fraîchement distillé, puis 75,6 mmol. d'alcool polyfluoré CF₃(CF₂)₆CH₂OH.

Puis on introduit sous atmosphère d'azote 2,15 g (90,7 mmol.) d'hydrure de sodium.

On maintient la suspension obtenue 30 minutes sous agitation à 20°C.

On introduit ensuite dans le ballon 14 ml (150 mmol.) de bromo-3 thiophène, puis 7 g (37 mmol.) d'iodure cuivreux.

Le ballon est maintenu à 90°C pendant 15 heures.

Puis le ballon est refroidi à 20°C.

La suspension refroidie est filtrée, reprise à l'éther puis lavée à l'eau.

La phase organique est séchée et évaporée sous vide.

Le produit est purifié par distillation sous vide (18 mmHg).

On obtient l'oxyde de 8,8,8,7,7,6,6,5,5,4,4,3,3,2,2-pentadécafluoro-n-octyle et de 3-thiényle avec un rendement de 71 % par rapport à l'alcool polyfluoré.

### Exemple 5 : Polymérisation de l'oxyde de 2,2,2-trifluoréthyle et de 3-thiényle. Préparation du polymère conducteur.

Comme réacteur, on utilise un ballon de 500 ml muni d'un robinet à trois voies, d'un agitateur, d'un thermomètre et d'un septum permettant d'y planter une aiguille reliée à une pompe doseuse.

Ce ballon est placé dans un bain thermostatique et est purgé par un cycle de 3 vides et de 3 rinçages à l'azote pur et sec.

Dans ce ballon, maintenu à -20°C sous azote, on introduit 80 ml de chloroforme et 22 g de chlorure ferrique anhydre.

On introduit ensuite dans ce milieu via la pompe doseuse 20 ml de chloroforme et 2,5 g de l'oxyde de 2,2,2-trifluoréthyle et de 3-thiényle tel qu'obtenu à l'exemple 1, le tout en 10 minutes.

Le ballon est ensuite maintenu sous agitation à -20°C pendant 2 heures, après quoi on centrifuge le milieu à 10000 tours par minutes (12000 G) pendant 5 minutes.

Le produit obtenu est lavé 5 fois avec 500 ml de chloroforme, centrifugé et séché sous vide dynamique à 20°C.

On obtient 2,55 g du polymère de l'oxyde de 2,2,2-trifluoréthyle et de 3-thiényle, polymère dopé qui présente une conductivité de 10⁻¹ S/cm.

1 g du produit obtenu est dissous dans 100 ml d'acétonitrile. La solution est étalée sur une plaque en verre fixée sur une tournette (vitesse de rotation : 250 tours par minute). Un film conducteur de l'ordre de 1 micron est ainsi obtenu.

### Exemple 6 : Polymérisation de l'oxyde de 2,2,2-trifluoréthyle et de 3-thiényle. Préparation du polymère.

Comme réacteur on utilise un ballon de 500 ml muni d'un robinet à trois voies, d'un agitateur, d'un thermomètre et d'un septum permettant d'y planter une aiguille reliée à une pompe doseuse.

Ce ballon est placé dans un bain thermostatique et est purgé par un cycle de 3 vides et de 3 rinçages à l'azote pur et sec.

Dans ce ballon, maintenu à -20°C sous azote, on introduit 80 ml de chloroforme et 22 g de chlorure ferrique anhydre.

On introduit ensuite dans ce milieu via la pompe doseuse 20 ml de chloroforme et 2,5 g de l'oxyde de 2,2,2-trifluoréthyle et de 3-thiényle tel qu'obtenu à l'exemple 1, le tout en 10 minutes.

Le ballon est ensuite maintenu sous agitation à -20°C pendant 2 heures, après quoi on introduit dans ce ballon, goutte à goutte, 100 ml d'eau déminéralisée.

Le produit obtenu est lavé 4 fois avec 50 ml d'eau, puis filtré et séché sous vide dynamique à 20°C.

On obtient 2,1 g du polymère de l'oxyde de 2,2,2-trifluoréthyle et de 3-thiényle, polymère dédopé qui présente une conductivité de l'ordre de 10⁻⁶ S/cm.

1,4 g du produit obtenu est dissous dans 20 ml de diméthylformamide. La solution est étalée sur une plaque en verre fixée sur une tournette (vitesse de rotation : 150 tours par minute pendant 2 minutes puis 250 tours par minute pendant 2 minutes). Le film obtenu est séché sous vide pendant 15 minutes à 20°C. Un film de l'ordre de 1 à 1,5 micron est ainsi obtenu.

### Exemple 7 : Polymérisation de l'oxyde de 4,4,4,3,3,2,2-heptafluoro-n-butyle et de 3-thiényle.

On utilise comme réacteur un ballon identique à celui décrit à l'exemple 5.

Dans ce ballon, maintenu à 5°C sous azote, on introduit 80 ml de chloroforme et 3,4 g de chlorure ferrique anhydre.

On introduit ensuite dans ce milieu via la pompe doseuse 20 ml de chloroforme et 1 g de l'oxyde de 4,4,4,3,3,2,2-heptafluoro-n-butyle et de 3-thiényle tel qu'obtenu à l'exemple 3, le tout en 10 minutes.

Le ballon est ensuite maintenu sous agitation à 5°C pendant 4 heures, après quoi on introduit 50 ml d'acétonitrile en 20 minutes.

Le produit est ensuite filtré sous azote.

Le produit obtenu est lavé 4 fois avec 50 ml d'acétonitrile, puis filtré et séché sous vide dynamique à 20°C.

On obtient 0,71 g du polymère de l'oxyde de 4,4,4,3,3,2,2-heptafluoro-n-butyle et de 3-thiényle, polymère dopé qui présente une conductivité de 10⁻³ S/cm avec un taux de conversion - polymère dopé sur monomère - égal à 71 %.

Ce polymère est soluble dans l'acétonitrile en donnant une solution bleue.

### Exemple 8 : Polymérisation de l'oxyde de 2,2,2-trifluoréthyle et de 3-thiényle.

La synthèse électrochimique du polymère et la voltampérométrie est réalisées avec un ensemble potentiostat-galvanostat équipé d'un générateur de signaux, d'un intégrateur et d'une table traçante. Des électrodes circulaires de 0,7 cm² en platine sont utilisées comme électrode de travail, un fil de platine est utilisé comme contre électrode et une électrode au calomel est utilisée comme électrode de référence.

La synthèse du polymère est effectuée dans une cellule électrochimique de 100 ml équipée d'une anode en platine poli de 0,7 cm² de surface, d'un fil de platine comme contre électrode et d'une électrode au calomel saturée en KCl (ESC) comme référence.

On introduit dans cette cellule 25 ml d'une solution du monomère (100 mmol.l⁻¹ de l'oxyde de 2,2,2-trifluoréthyle et de 3-thiényle) et de l'hexafluorophosphate de tétrabutylammonium (50 mmol.l⁻¹) dans du chlorure de méthylène.

La cellule est purgée à l'azote et est reliée à l'ensemble potentiostat-galvanostat décrit ci-dessus.

On procéde à une polymérisation sous conditions galvanostatiques en imposant un courant de 4 mA/cm². Les dépôts de polymère sont réalisés à 20°C, sous atmosphère d'azote, après dégazage de la solution par barbotage d'azote.

Le polymère est déposé sur une électrode de platine d'une surface de 0,7 cm² polie. La quantité de charges utilisées est de 200 mC/cm² et la densité de courant est égale à 4 mA/cm².

Le polymère obtenu est partiellement soluble dans le milieu de synthèse et soluble dans l'acétone et le chloroforme notamment.

Les propriétés électrochimiques du polymère ont été mesurées à partir du voltammogramme cyclique enregistré au moyen d'un potentiostat et à partir des pics d'intensité enregistrés. La figure 1 représente un voltammogramme réalisé avec une vitesse de balayage de 50 mV/s. L'unité de l'abscisse est le volt (V), l'unité de l'ordonnée est le microampère (µA).

L'électrode avec le dépôt de polymère est rincée à l'acétone, sechée à l'air comprimée, puis plongée dans une cellule électrochimique contenant une solution de 100 mmol/l d'hexafluorophosphate de tétrabutylammonium dans du chlorure de méthylène.

La cellule est purgée à l'azote.

Le polymère est soumis à des potentiels situés entre -0,05 et +0,8 V/ESC. La charge du courant d'oxydation est de 7,1 mC/cm². Le potentiel du pic anodique (EPA) est de 0,61 V/ESC. Le taux de dopage derivé est égal à 7 %.

### Exemple 9 : Polymérisation de l'oxyde de 8,8,8,7,7,6,6,5,5,4,4,3,3,2,2-pentadécafluoro-n-octyle et de 3-thiényle

La synthèse électrochimique et la voltampérometrie sont réalisées avec un ensemble tel que décrit à l'exemple 8. La synthèse du polymère est effectuée dans une cellule électrochimique de 100 ml équipée d'une anode en platine poli de 0,7 cm² de surface, d'un fil de platine comme contre électrode et d'une électrode en calomel saturée en KCl (ESC) comme référence. On introduit dans cette cellule 25 ml (100 mmol.l⁻¹) d'une solution de l'oxyde de 8,8,8,7,7,6,6,5,5,4,4,3,3,2,2-pentadécafluoro-n-octyle et de 3-thiényle tel qu'obtenu à l'exemple 4, et de perchlorate de lithium (50 mmol.l⁻¹) dans de l'acétonitrile fraîchement distillé.

La cellule est purgée à l'azote et est reliée à l'ensemble potentiostat-galvanostat décrit à l'exemple 8. On procède à une polymérisation sous conditions galvanostatiques en imposant un courant de 1,9 mA/cm². Les dépôts de polymères sont réalisés à 20°C sous atmosphère d'azote. La quantité de charge utilisée est de 29,3 mc/cm², ce qui conduit à un dépôt très fin de polymère.

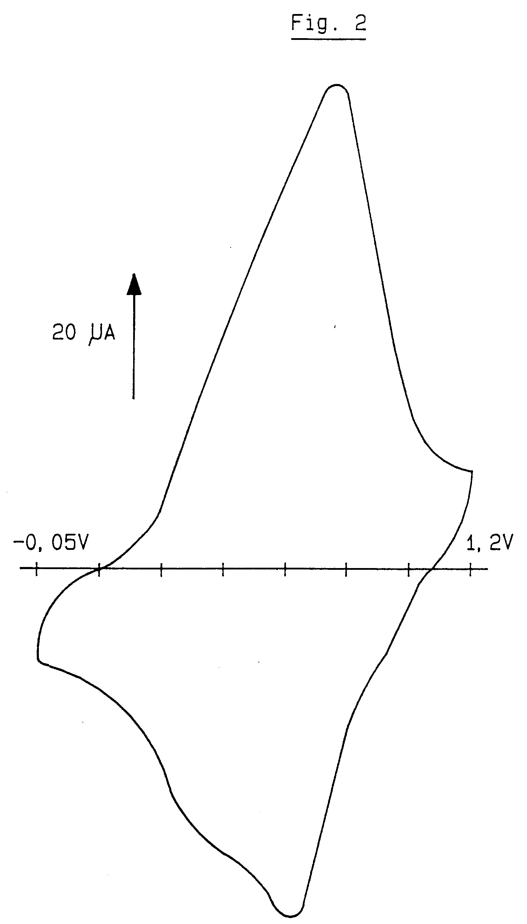

Les propriétés électrochimiques du polymère ainsi obtenu ont été mesurées à partir du voltampérogramme cyclique enregistré au moyen d'un potentiostat et à partir des pics d'intensité enregistrés. La figure 2 représente un voltampérogramme réalisé avec une vitesse de balayage de 50 mV/s. L'unité de l'abscisse est le volt (V), l'unité de l'ordonnée est le microampère (µA).

L'électrode avec le dépôt de polymère est rincée à l'acétone, sechée à l'air comprimé, puis plongée dans une cellule électrochimique contenant une solution de 100 mmol/l de perchlorate de lithium dans l'acétonitrile.

La cellule est purgée à l'azote.

Le polymère est soumis à une variation de potentiels situés entre -0,05 et +1,25 V/ESC. La charge du courant d'oxydation est de 2,81 mC/cm².

Le potentiel du pic anodique (EPA) est de 0,87 V/ESC. Le potentiel du pic cathodique (EPC) est de 0,70 V/ESC. Le taux de dopage derivé est égal à 21,2 %.

Parmi les propriétés intéressantes de ce polymère, on peut citer sa stabilité sous cyclage. Un dépôt de 100 mc/cm² de polymère sur une électrode en platine de 0,7 cm² de surface est soumis à un balayage du potentiel entre -0,2 et 1,15 V/ECS à une vitesse de 200 mV/s.

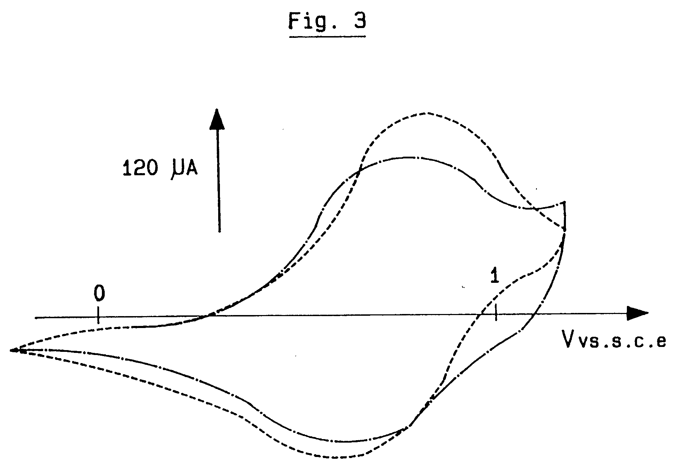

Après 3000 cycles, ce film échange toujours 70 % de sa charge initiale, code le montre la figure 3.

### Exemple 10

On utilise une cellule électrochimique de 100 mm de longueur, 12 mm de largeur et 40 mm de hauteur, équipée de 3 électrodes :
- l'anode est constituée d'une lame de verre recouverte d'oxyde d'étain et d'indium de 10 mm de large, la partie immergée étant de 2 cm²,
- la cathode est constituée par une lame de platine,
- l'électrode de référence est saturée au calomel.

On introduit dans cette cellule une solution de 25 cm³ de l'oxyde 8,8,8,7,7,6,6,5,5,4,4,3,3,2,2-pentadécafluoro-n-octyle et de 3-thiényle tel qu'obtenu à l'exemple 4, et de perchlorate de lithium (50 mmol.l⁻¹) dans de l'acétonitrile fraîchement distillé.

La cellule est purgée à l'azote et est reliée à l'ensemble potentiostat-galvanostat décrit à l'exemple 8. On procède à une polymérisation sous conditions galvanostatiques en imposant un courant de 1,9 mA/cm². On couvre la cellule avec une plaque de verre. Les dépôts de polymères sont réalisés à 20°C sous atmosphère d'azote.

On aboutit à un dépôt total de l'ordre de 4 microns d'épaisseur.

La conductivité mesurée par la méthode des quatre points est de l'ordre de 5 S.cm⁻¹.

## Revendications

1. Thiophènes substitués de formule générale : 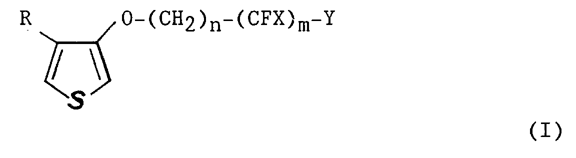 dans laquelle :
- R représente un atome d'hydrogène, un atome d'halogène, ou un groupement aliphatique contenant de 1 à 4 atomes de carbone,
- X représente un atome d'hydrogène ou un atome d'halogène,
- Y représente un atome d'hydrogène, un atome d'halogène, un groupement aliphatique linéaire ou non, non substitué ou substitué par un ou plusieurs atomes d'halogène et contenant de 1 à 6 atomes de carbone ou un groupement aromatique non substitué ou substitué par un ou plusieurs atomes d'halogène,
- n représente un nombre entier égal ou supérieur à 1,
- m représente un nombre entier égal ou supérieur à 1.

2. Thiophènes substitués selon la revendication 1 caractérisés en ce que
- R représente un atome d'hydrogène,
- X représente un atome de fluor,
- Y représente un atome d'hydrogène, un atome de fluor ou un radical -CF₃,
- n représente un nombre entier tel que 1 ≤ n ≤ 6,
- m représente un nombre entier tel que 1 ≤ m ≤ 14.

3. Thiophènes substitués selon la revendication 2 caractérisés en ce que
- Y représente un atome de fluor,
- n représente un nombre entier égal à 1,
- m représente un nombre entier égal à 1, 3 ou 7.

4. Procédé pour la fabrication de thiophènes substitués selon l'une des revendications 1 à 3, caractérisé en ce que l'on fait réagir un composé de formule générale 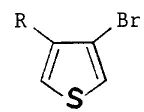 avec un composé de formule générale [O-(CH₂)ₙ-(CFX)ₘ-Y]⁻ en présence de K⁺ ou Na⁺, CuI et d'un solvant.

5. Polymères substitués de formule générale : 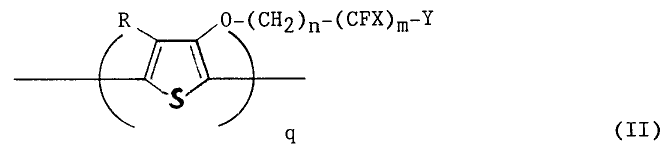 dans laquelle :
- q représente un nombre entier,
- R représente un atome d'hydrogène, un atome d'halogène, ou un groupement aliphatique contenant de 1 à 4 atomes de carbone,
- X représente un atome d'hydrogène ou un atome d'halogène,
- Y représente un atome d'hydrogène, un atome d'halogène, un groupement aliphatique linéaire ou non, non substitué ou substitué par un ou plusieurs atomes d'halogène et contenant de 1 à 6 atomes de carbone ou un groupement aromatique non substitué ou substitué par un ou plusieurs atomes d'halogène,
- n représente un nombre entier égal ou supérieur à 1,
- m représente un nombre entier égal ou supérieur à 1.

6. Polymères substitués selon la revendication 5, caractérisés en ce que :
- q représente un nombre entier compris entre 2 et 1000,
- R représente un atome d'hydrogène,
- X représente un atome de fluor,
- Y représente un atome d'hydrogène, un atome de fluor ou un radical -CF₃,
- n représente un nombre entier tel que 1 ≤ n ≤ 6,
- m représente un nombre entier tel que 1 ≤ m ≤ 14.

7. Polymères substitués selon la revendication 6 caractérisés en ce que
- q représente un nombre entier compris entre 2 et 500,
- Y représente un atome de fluor,
- n représente un nombre entier égal à 1,
- m représente un nombre entier égal à 1, 3 ou 7.

8. Polymères conducteurs d'électricité contenant un polymère selon l'une des revendications 5 à 7 et un agent dopant.

9. Procédé pour la préparation de polymères conducteurs d'électricité selon la revendication 8 caractérisé en ce que l'on polymérise chimiquement, dans un milieu réactionnel comprenant un oxydant et un solvant du thiophène substitué, un monomère choisi parmi les thiophènes substitués selon l'une des revendications 1 à 3.

10. Procédé pour la préparation de polymères conducteurs d'électricité selon la revendication 8 caractérisé en ce que l'on polymérise électrochimiquement, par oxydation anodique au sein d'un solvant polaire et en présence d'électrolytes appropriés, un monomère choisi parmi les thiophènes substitués selon l'une des revendications 1 à 3.

11. Procédé pour la préparation de polymères selon l'une des revendications 5 à 7, caractérisé en ce que l'on mélange avec de l'eau ou un alcool un polymère selon la revendication 8.

12. Utilisation des polymères conducteurs selon la revendication 8 ou des polymères selon l'une des revendications 5 à 7 sous forme de couches épaisses ou minces, au travers ou non de masques sérigraphiques, ou sous forme de films d'épaisseur inférieure au micron sur divers supports tels que verre, verre métallisé, métaux, textiles.

13. Dispositifs électroconducteurs contenant un polymère conducteur d'électricité selon la revendication 8.

## Claims

1. Substituted thiophenes of general formula : 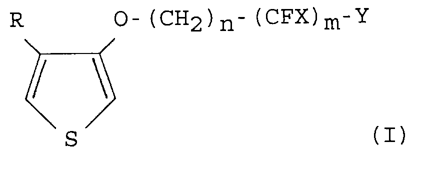 in which
- R represents a hydrogen atom, a halogen atom or an aliphatic group containing from 1 to 4 carbon atoms,
- X represents a hydrogen atom or halogen atom,
- Y represents a hydrogen atom, a halogen atom, a straight-chain or branched aliphatic group which is unsubstituted or substituted by one or more halogen atoms and contains from 1 to 6 carbon atoms or an aromatic group which is unsubstituted or substituted by one or more halogen atoms,
- n represents an integer equal to or greater than 1, and
- m represents an integer equal to or greater than 1.

2. Substituted thiophenes according to Claim 1, characterised in that
- R represents a hydrogen atom,
- X represents a fluorine atom,
- Y represents a hydrogen atom, a fluorine atom, or a -CF₃ radical,
- n represents an integer such that 1 ≤ n ≤ 6
- m represents an integer such that 1 ≤ m ≤ 14.

3. Substituted thiophenes according to Claim 2, characterised in that
- Y represents a fluorine atom,
- n represents an integer equal to 1,
- m represents an integer equal to 1, 3 or 7.

4. Process for the production of substituted thiophenes according to any one of Claims 1 to 3, characterised in that a compound of general 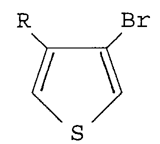 is reacted with a compound of general formula [O-(CH₂)ₙ-(CFX)ₘ-Y]⁻ in the presence of K⁺ or Na⁺, CuI and a solvent.

5. Substituted polymers of general formula : 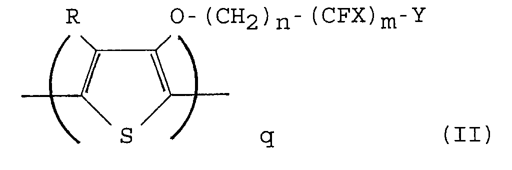 in which :
- q represents an integer,
- R represents a hydrogen atom, a halogen atom or an aliphatic group containing from 1 to 4 carbon atoms,
- X represents a hydrogen atom or a halogen atom,
- Y represents a hydrogen atom, a halogen atom, a straight-chain or branched aliphatic group which is unsubstituted or substituted by one or more halogen atoms and contains from 1 to 6 carbon atoms or an aromatic group which is unsubstituted or substituted by one or more halogen atoms,
- n represents an integer equal to or greater than 1, and
- m represents an integer equal to or greater than 1.

6. Polymers according to Claim 5, characterised in that
- q represents an integer of between 2 and 1000,
- R represents a hydrogen atom,
- X represents a fluorine atom,
- Y represents a hydrogen atom, a fluorine atom, or a -CF₃ radical,
- n represents an integer such that 1 ≤ n ≤ 6
- m represents an integer such that 1 ≤ m ≤ 14.

7. Polymers according to Claim 6, characterised in that
- q represents an integer of between 2 and 500.
- Y represents a fluorine atom,
- n represents an integer equal to 1, and
- m represents an integer equal to 1, 3 or 7.

8. Electrically conductive polymers containing a polymer according to any one of Claims 5 to 7 and a doping agent.

9. Process for the preparation of electrically conductive polymers according to Claim 8, characterised in that a monomer chosen from the substituted thiophenes according to any one of Claims 1 to 3 is chemically polymerised in a reaction medium comprising an oxidising agent and a solvent for the substituted thiophene.

10. Process for the preparation of electrically conductive polymers according to Claim 8, characterised in that a monomer chosen from the substituted thiophenes according to any one of Claims 1 to 3 is electrochemically polymerised by anodic oxidation within a polar solvent and in the presence of appropriate electrolytes.

11. Process for the preparation of polymers according to any one of Claims 5 to 7, characterised in that a polymer according to Claim 8 is mixed with water or an alcohol.

12. Use of the conductive polymers according to Claim 8 or of the polymers according to any one of Claims 5 to 7 in the form of thick or thin layers, optionally through serigraphic masks, or in the form of films having a thickness of less than one micron on various supports such as glass, metallised glass, metals or textiles.

13. Electroconductive devices containing an electrically conductive polymer according to Claim 8.

## Patentansprüche

1. Substituierte Thiophene der allgemeinen Formel 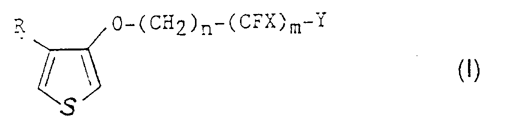 worin:
- R ein Wasserstoffatom, ein Halogenatom oder eine aliphatische Gruppe mit 1 bis 4 Kohlenstoffatomen darstellt,
- X ein Wasserstoffatom oder ein Halogenatom darstellt,
- Y ein Wasserstoffatom, ein Halogenatom, eine lineare oder nicht lineare, nicht substituierte oder mit einem oder mehreren Halogenatomen substituierte aliphatische Gruppe mit 1 bis 6 Kohlenstoffatomen oder eine nicht substituierte oder mit einem oder mehreren Halogenatomen substituierte aromatische Gruppe darstellt,
- n eine ganze Zahl gleich oder größer 1 darstellt,
- m eine ganze Zahl gleich oder größer 1 darstellt.

2. Substituierte Thiophene gemäß Anspruch 1, dadurch gekennzeichnet, daß
- R ein Wasserstoffatom darstellt,
- X ein Fluoratom darstellt,
- Y ein Wasserstoffatom, ein Fluoratom oder einen -CF₃-Rest darstellt,
- n eine solche ganze Zahl darstellt, daß 1 ≤ n ≤ 6,
- m eine solche ganze Zahl darstellt, daß 1 ≤ m ≤ 14.

3. Substituierte Thiophene gemäß Anspruch 2, dadurch gekennzeichnet, daß
- Y ein Fluoratom darstellt,
- n eine ganze Zahl gleich 1 darstellt,
- m eine ganze Zahl gleich 1, 3 oder 7 darstellt.

4. Verfahren zur Herstellung von substituierten Thiophenen gemäß einem der Ansprüche 1 bis 3, dadurch gekennzeichnet, daß man eine Verbindung der allgemeinen Formel 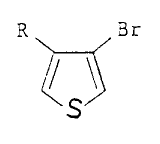 mit einer Verbindung der allgemeinen Formel [O-(CH₂)ₙ-(CFX)ₘ-Y]⁻ in Gegenwart von K⁺ oder Na⁺, CuI und einem Lösungsmittel umsetzt.

5. Substituierte Polymere der allgemeinen Formel 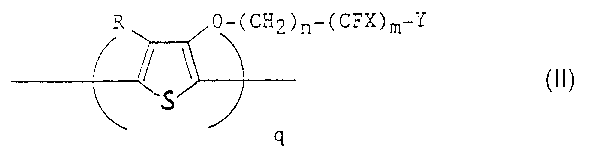 worin:
- q eine ganze Zahl darstellt,
- R ein Wasserstoffatom, ein Halogenatom oder eine aliphatische Gruppe mit 1 bis 4 Kohlenstoffatomen darstellt,
- X ein Wasserstoffatom oder ein Halogenatom darstellt,
- Y ein Wasserstoffatom, eine Halogenatom, eine lineare oder nicht lineare, nicht substituierte oder mit einem oder mehreren Halogenatomen substituierte aliphatische Gruppe mit 1 bis 6 Kohlenstoffatomen oder eine nicht substituiere oder mit einem oder mehreren Halogenatomen substituierte aromatische Gruppe darstellt
- n eine ganze Zahl gleich oder größer 1 darstellt,
- m eine ganze Zahl gleich oder größer 1 darstellt.

6. Substituierte Polymere gemäß Anspruch 5, dadurch gekennzeichnet, daß
- q eine ganze Zahl zwischen 2 und 1000 darstellt,
- R ein Wasserstoffatom darstellt,
- X ein Fluoratom darstellt,
- Y ein Wasserstoffatom, ein Fluoratom oder einen -CF₃-Rest darstellt,
- n eine solche ganze Zahl darstellt, daß 1 ≤ n ≤ 6,
- m eine solche ganze Zahl darstellt, daß 1 ≤ m ≤ 14.

7. Substituierte Polymere gemäß Anspruch 6, dadurch gekennzeichnet, daß
- q eine ganze Zahl zwischen 2 und 500 darstellt,
- Y ein Fluoratom darstellt,
- n eine ganze Zahl gleich 1 darstellt,
- m eine ganze Zahl gleich 1, 3 oder 7 darstellt.

8. Elektrisch leitfähige Polymere, die ein Polymer gemäß einem der Ansprüche 5 bis 7 und ein Dotierungsmittel enthalten.

9. Verfahren zur Herstellung elektrisch leitfähiger Polymere gemäß Anspruch 8, dadurch gekennzeichnet daß man ein unter den substituierten Thiophenen gemäß einem der Ansprüche 1 bis 3 ausgewähltes Monomer in einem Reaktionsmedium, das ein Oxidationsmittel und ein Lösungsmittel für das substituierte Thiophen umfaßt, chemisch polymerisiert.

10. Verfahren zur Herstellung elektrisch leitfähiger Polymere gemäß Anspruch 8, dadurch gekennzeichnet, daß man ein unter den substituierten Thiophenen gemäß einem der Ansprüche 1 bis 3 ausgewähltes Monomer durch anodische Oxidation in einem polaren Lösungsmittel und in Gegenwart geeigneter Elektrolyten elektrochemisch polymerisiert.

11. Verfahren zur Herstellung von Polymeren gemäß einem der Ansprüche 5 bis 7, dadurch gekennzeichnet, daß man ein Polymer gemäß Anspruch 8 mit Wasser oder einem Alkohol mischt.

12. Verwendung der leitfähigen Polymere gemäß Anspruch 8 oder der Polymere gemäß einem der Ansprüche 5 bis 7 in Form von dicken oder dünnen Schichten durch serigraphische Masken hindurch oder nicht hindurch oder in Form von Filmen mit einer Dicke im Submikrometer-Bereich auf verschiedenen Trägern wie Glas, metallisiertem Glas, Metall, Textilien.

13. Elektrisch leitende Vorrichtungen, die ein elektrisch leitfähiges Polymer gemäß Anspruch 8 enthalten.
